# EUROPEAN PATENT APPLICATION

(11) **EP 0 779 360 A1**
(43) Date of publication of application: **18.06.1997**
(21) Application number: 95928623.8
(22) Date of filing: 21.08.1995
(51) Int. Cl.: C12N 5/22, C12N 15/07, C12N 15/24, C12Q 1/68, C12Q 1/66

(54) **HYBRIDOMA THAT EXPRESSES mRNA OF INTERLEUKIN-5 AND METHOD OF SCREENING DRUGS THEREWITH**

(30) Priority: 19.08.1994 JP 195031/94
(71) Applicant: Environmental Research Institute, Inc., Tokyo 141 (JP)
(72) Inventor: OKUDAIRA, Hirokazu, Tokyo 112 (JP); MORI, Akio, Bunkyo-ku Tokyo 113 (JP); MIKAMI, Tadashi, Chiba 287 (JP)
(74) Representative: VOSSIUS & PARTNER
(86) International application number: JP9501647
(87) International publication number: WO9606162

(57) **Abstract**

A method for screening an antiasthmatic or antiallergic drug, which comprises activating a hybridoma capable of expressing mRNA of interleukin-5 (IL-5) but not producing IL-5 in the presence of a compound or substance to be tested and observing whether the expression of the IL-5 gene takes place, said hybridoma being obtainable by fusing a human T-cell clone expressing IL-5 mRNA with a cancered T-cell.

## Description

### Technical Field

The present invention relates to a hybridoma expressing mRNA of interleukin-5 (hereinafter referred to as "IL-5") and a method for screening drugs (particularly antiasthmatic and antiallergic drugs) using the hybridoma.

### Background Art

Treatment of asthmatic or allergic diseases and development of drugs therefor have heretofore been made using animal models such as mice, rats, guinea pigs and the like. In recent years, the progress of cellular immunology and molecular biology has made it possible to carry out the study in the related art field at the level of cells. For instance, measurement and study on various chemical mediators called chemokines, cytokines and the like at the level of cells are reported [e.g., Hirokuni Otsuka, "Yakuri to Chiryo (Pharmacology and Therapy)", 20, 4081-4087 (1992); Naomi Matsuda, "Yakuri to Chiryo (Pharmacology and Therapy)", 21, 1475-1477 (1993); Reiji Okazaki, "Shinyaku to Rinshou (New Drugs and Clinics)", 42, 2092-2101 (1993)].

On the other hand, it has been suggested that IL-5, which is a cytokine produced or secreted by T cells (T lymphocytes), would be available as an indicator in the study on treatment of asthma caused by airway contraction mediated eosinophil leucocytes, delayed type allergic reaction and atopic dermatitis and on drugs to be used for such treatment [H.Okudaira et. al., ACI News, 6/1 (1994); A. Mori et al., International Archives of Allergy and Immunology, 104, 32-35 (1994)]. It has also been reported that T cells secrete IL-5 and express IL-5 mRNA (IL-5 gene) [e.g., Hugh D. Campbell et al., Proceedings of the National Academy of Sciences of the United States of America, 84, 6629-6633 (1987)].

IL-5 is a water-soluble immunomodulator of 12,500 in molecular weight as produced and secreted by T cells, which has the following characteristic properties:
a) converting activated B-cells into antibody-forming cells;
b) promoting production of leukotriene C4 and others by eosinophil leukocytes;
c) promoting degranulation of eosinophil leukocytes;
d) promoting differentiation and proliferation of eosinophil leukocytes;
e) enhancing mobility (chemotaxis) of eosinophil leukocytes to PAF, leukotriene B4 and FMLP by preculture; and
f) increasing adhesion of eosinophil leukocytes to microvasculature endothelium.

From the above, it is understood that IL-5 participates in differentiation and proliferation of eosinophil leukocytes, particularly in allergic crisis among various immune responses to a considerable extent. Accordingly, testing and studying with a system mediating IL-5 is considered to be meaningful for establishment of a method for treatment of asthma, delayed type allergic reaction, atopic dermatitis, etc. as well as development of drugs for such treatment, and in particular, the performance of screening for development of new drugs with said system would be very simple and highly reliable [A. Mori et. al., Environmental Dermatology, 1, 42-54 (1994); A. Mori et. al., International Archives of Allergy and Immunology, 104, 32-35 (1994)].

Human T cell clones secreting IL-5 were already established and can express mRNA of IL-5 [e.g., Akio Mori, Abstract of the 43rd Meeting of Japan Allergy Society, p 1334 (1994)]. In general, however, these T cell clones as established cannot be cultured over a long period of time, and their maintenance is thus hardly possible. Production of IL-5 comprises expression of an IL-5 gene in T cells and secretion of expressed IL-5. Such IL-5 production can be observed in said T-cell clones, but these T-cell clones are not suitable for screeing in development of drugs, because of the difficulty in their maintenance as stated above. For this reason, establishment of T-cell clones which can be cultured permanently has been desired.

### Technical Problem To Be Solved By Invention

An object of the present invention is to provide T-cell clones which can be maintained over a long period of time and enable expression of an IL-5 gene. Another object of the invention is to provide a method for effectively screening antiasthmatic and antiallergic drugs using such T-cell clones.

### Solution of Technical Problem

As the result of an extensive study, the present inventors have succeeded in obtaining from hyman T-cell clones and cancered T-cells hybridomas which enable expression of IL-5 mRNA without production of IL-5, can be proliferated and are treated with ease. Due to these characteristic properties, said hybridomas can be used as an indicator for the control of expression of IL-5 mRNA in screening antiasthmatic and antiallergic drugs at the level of cells.

The hybridoma of this invention is obtainable by fusing a human T cell clone expressing IL-5 mRNA with a cancered T cell. Such hybridoma can express IL-5 mRNA but do not produce IL-5. It can be used itself in screening for antiasthmatic and antiallergic drugs, but introduction of a plasmid vector incorporated with a gene encoding a proper enzyme and a transcriptional control region of an IL-5 gene to said hybridoma enables to determine the expression of IL-5 mRNA indirectly by measurement of the enzymatic activity whereby desired screening can be accomplished more easily.

For preparation of the hybridoma of the invention, T-cell clones are first established, and then clones expressing IL-5 mRNA are selected from them. The selected clones are then fused with cancered T cells as separately cultured to obtain hybridomas, from which clones expressing IL-5 mRNA are selected. The above preparation may be carried out specifically as described below.

### (1) Establishment of T-cell clones

T-cell clones may be established, for instance, by the limiting dilution method as described on "Zoku-Seikagaku Jikken Koza (Sequel to Biochemistry Experimental Course)", Vol. 5, Immunobiochemistry Research Method, Tokyo Kagakudojin, page 206. That is, a sample of peripheral blood is collected from an allergic patient, and a lymphocyte-containing fraction is separated and cultured in a serum-free medium. After about two weeks, a lymphocyte-containing fraction is likewise separated from the peripheral blood sample collected from the same allergic patient as above and then irradiated with X-ray to give antigen-presenting cells. Subsequently, the cultured cells are subjected to limiting dilution using a serum-free medium mixed with an antigen and the antigen-presenting cells as described above. After about 2 weeks, the intended T-cell clones are established by culturing only single colonies.

### (2) Observation on expression of IL-5 mRNA in T-cells

The T-cell clones as obtained in (1) are cultured on a well-type plate and stimulated by calcium ionophore. Thereafter, the T-cell clones are collected and centrifuged, and after aspiration of the supernatant, an RNA extract (ISOGEN, manufactured by Nippon Gene Co.) is added thereto, followed by stirring. The resulting mixture is treated with chloroform, ethanol or the like and dried. The dried product is dissolved in water and treated with a reverse transcriptase-containing RT reaction solution as shown in Table 1 and the like to give cDNA, followed by subjecting to the PCR method. Thereafter, a dyestuff is added to each sample and subjected to electrophoresis for confirmation of the production of IL-5 cDNA. The T-cell clones corresponding to the samples on which the production is confirmed are selected.

### (3) Culture of cancered T-cells

Among these cells, BUC cells show resistance to 8-azaganine resistance, and hybridomas fused in a HAT medium can be selected, for instance, by the method as described in "Zoku-Seikagaku Jikken Koza (Sequel to Biochemistry Experimental Course)", Vol. 5, Immunobiochemistry Research Method, Tokyo Kagakudojin, p. 262. The BUC cells can be cultured in a RPMI 1640 medium containing 10 % FCS (fetal calf serum) or the like.

### (4) Production of hybridoma

Fusion may be carried out by the method as described in "Zoku-Seikagaku Jikken Koza (Sequel to Biochemistry Experimental Course)". Vol.5, Immunobiochemistry Research Method, Tokyo Kagakudojin, p. 262, with slight modification. However, if the fusion is carried out without stimulation of T cell clones as obtained in (2), no desired hybridoma is produced. It is therefore necessary to use a method wherein T-cell clones are stimulated. For instance, the desired hybridoma is obtainable by fusion with oncogenic (malignant or myeloma) T cells by the aid of polyethylene glycol after PMA (phorbol 12-myristate 13-acetate) + IOM (ionomycin) stimulation (PMA, 20 nM; IOM, 1 uM).

### (5) Selection of clones expressing IL-5 mRNA

Selection may be carried out in the same manner as in (2).

Use of the hybridomas obtained thus make it possible to conduct the screening for antiasthmatic and antiallergic drugs, as utilized them as an indicator of expression of IL-5 mRNA. The method for screening may comprise, for instance, adding a test compound or substance to the hybridoma of the invention and subjecting the mixture to treatment for conversion into IL-5 cDNA in the same manner as in (2), followed by the PCR method, electrophoresis, etc. to confirm the production of IL-5 cDNA.

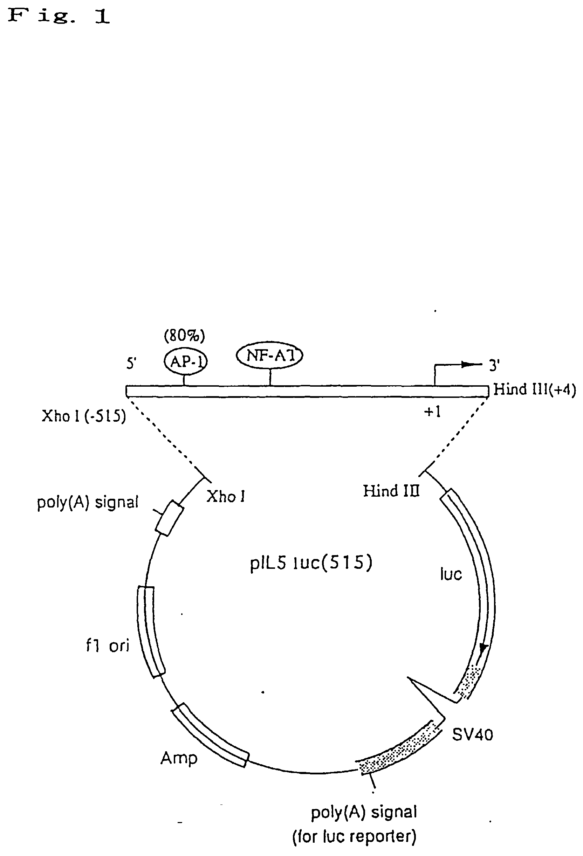

Alternatively, the hybridoma of the invention may be transformed with a plasmid into which a proper enzyme gene and the transcriptional control region of an IL-5 gene are incorporated (cf. Fig. 1 of the attached drawing). By means of this transformed hybridoma, screening for antiasthmatic and antiallergic drugs can be made through measurement of the enzymatic activity. The enzyme usable in this invention includes luciferase, β-galactosidase, chloramphenicol acetyl transferase and the like. In particular, luciferase is preferred. Introduction of the gene into the cell may be carried out by a per se conventional procedure such as the calcium phosphate method or the electroporation method ["Saibo Kougaku Zikken Protocol (Cell Engineering Experiment Protocol)" (Shujunsha), p. 212-223 (1993)].

The method of this invention using the hybridoma into which an enzyme gene is incorporated can determine the test result by measurement of the enzymatic activity and therefore enables to obtain a more definite outcome in a simpler operation in comparison with the method wherein measurement is effected through the step of extraction of IL-5 mRNA. In the method of the inveniton, it is thus possible to predict the effectiveness of a test comound or substance for such diseases as asthma, delayed type allergic reaction and atopic dermatitis through observing whether the expression of the enzymatic activity (e.g., luciferase activity) is prevented by the test compound or substance.

Cloning of human IL-5 gene itself has already been achieved, and analysis of the AP-1 (activating protein 1) and NF-AT (nuclear factor of activated T-cells) binding regions has also been accomplished. In comparison with interleukin 2 (IL-2), the AP-1 binding region is considered to have a homology of about 80 %, while the NF-AT binding region is considered to have nearly 100 % homology [e.g., see Hugh D. Cambell et. al., Proceedings of the National Academy of Sciences of the United States of America, 84, 6629-6633 (1987), T. Tanabe et. al., The Journal of Biological Chemistry, 262, 16580-16584 (1987), H. Okudaira et. al., ACI News, 6/1 (1994)].

By the use of the hybridoma of the invention, it is possible to determine whether or not the test compound or substance acts on the NF-AT binding region. It seems that FK506 known as an immunosuppressive agent acts on the NF-AT binding region to suppress the production of IL-5 [A. Mori et. al., International Archives of Allergy and Immunology, 104, 32-35 (1994)]. Also, dexamethasone (DX) as a kind of glucocorticoid is known to inhibit the synthesis of the AP-1 protein and eventually considered to inhibit the action of AP-1 onto the AP-1 binding region, whereby the production of IL-5 is suppressed. The plasmid vector as prepared according to this invention includes the AP-1 binding region in about 80 %, yet suppression of the luciferase activity by addition of DX was not produced. From this fact, a perfect AP-1 binding region may be considered to exist in the upperstream of the gene sequence.

### Technical Effect of Invention:

The hybridoma of the invention does not produce IL-5, while it can express IL-5 m-RNA. Also, it shows good proliferation and its properties are not changed even after if it is thawed after cryopreservation or lyophilization. In addition, it can be easily handled or treated. The culture system of the hybridoma according to the invention can be used for observation and study of the preventive effect of a test compound or substance on the IL-5 gene expression, by which the drugs on such diseases as asthma, delayed type allergic reaction and atopic dermatitis may be screened.

The present invention will be hereinafter explained more in detail with reference to examples which however should not be understood to restrict the technical scope of this invention.

### Examples

### Example 1: Production of hybridoma

### (1) Establishment of T-cell clones

Establishment of T-cell clones was carried out by the limiting dilution method as described in "Zoku-Seikagaku Jikken Koza (Sequel to Biochemistry Experimental Course)", Vol.5, Immunobiochemistry Research Method (Tokyo Kagakudoujin), p.206. That is, a sample of peripheral blood was collected from an allergic patient, and a lymphocyte-containing fraction was separated by the aid of Ficoll, followed by washing twice with saline. Then, the cells were cultured in a serum-free medium (AIM-V, manufactured by GIBCO Co.) containing IL-2 20 U/ml. About two weeks later, a lymphocyte-containing fraction was likewise separated from the peripheral blood sample collected from the same allergic patient as above and irradiated with X-ray (2,500 rad) to obtain antigen-presenting cells. The above cultured cells were subjected to limiting dilution by the use of a serum-free medium (AIM-V) mixed with an antigen (mites, etc.) and the antigen-presenting cells. About two weeks later, the mixture was subjected to microscopic observation, and only single colonies were cultured to establish T-cell clones. The thus obtained T cell clones were successively cultured by addition of the antigen-presenting cells thereto about every two weeks, said antigen-presenting cells being the ones prepared from the peripheral blood of the allergic patient from which said T cell clones were originated.

### (2) Observation on expression of IL-5 mRNA in T-cells

For selection of the clones suitable for cell fusion, i.e. clones expressing IL-5 mRNA, from the T cell clones prepared as in (1), mRNA expression was examined. Namely, the T cell clones were cultured in a 24 well plate (manufactured by CORNING Co.) at a concentration of 2 × 10⁶ cell/well and stimulated by calcium ionophore (PMA, 20 nM; IOM, 1 µM). Six hours later, the T cell clones were collected and centrifuged by a cooling centrifuge (KUBOTA 1710) at 4,000 rpm for 10 minutes at 4°C. After aspirating the supernatant, 1 ml of ISOGENE (manufactured by Nippon Gene Co.) was added, followed by stirring well. Homogenization was made 10 times by the aid of a 5 ml injection cylinder euipped with a 25G needle. Two hundred µl of chloroform was added thereto, stirred well and centrifuged at 13,000 rpm for 15 minutes at 4°C. The supernatant (aqueous layer) was collected, and 500 µl of isopropanol was added thereto. After stirring, the resultant mixture was maintained at -20°C for 1 hour or more. After centrifugation at 13,000 rpm for 15 minutes at 4°C, the solid material was collected by decantation, 1 ml of 80 % ethanol was added thereto, and stirring was carried out. Then, centrifugation was effected at 13,000 rpm for 15 minutes at 4°C, and decantation was made to collect a sodil material. The solid material was dried at room temperature, and 400 µl of TE buffer, 40 µl of a 3M sodium acetate solution (pH 5.8) and 1 ml of 100 % ethanol were added thereto, followed by stirring well and keeping at -20°C for one hour or more. After centrifugation at 13,000 rpm for 15 minutes at 4°C, decantation was carried out to collect a solid material, which is dried at room temperature and dissolved in 10 µl of distilled water. Incubation was carried out at 70°C for 4 minutes, followed by cooling on ice. To 10.5 µl of the resultant solution, 9.5 µl of the RT reaction solution as shown in Table 1 was added, followed by reacting at 42°C for 3 hours to transform to cDNA. After completion of the reaction, the PCR reaction solution as shown in Table 2 was prepared, and 45 µl of such solution was added to 5 µl of the cDNA-containing solution. Then, 33 µl of light mineral oil (manufactured by Sigma Co.) was added thereto, and the PCR method was carried out under the protocol as shown in Table 3. After completion of the PCR method, 2 µl of Type III pigment was added to 10 µl of the resulting solution, and electrophoresis was carried out with 1 % gel. Then, the band on electrophoresis was observed to select the T cell clones for establishing the hybridoma expressing IL-5 mRNA.

**Table 1**

| | |
|---|---|
| 5 x reaction buffer | 4 µl |
| 0.1 M DDT | 2 µl |
| 40 U/µl RNasin | 1 µl |
| 10mM dNTPmix | 1 µl |
| random primer | 1.5 µl |
| riverse transcriptase | 1 µl |
| sample | 9.5 µl |
| Total | 20 µl |

**Table 2**

| | |
|---|---|
| 10 x PCR reaction buffer | 4 µl |
| 2.5 mM MgCl₂ | 3 µl |
| sterile water | 32.6 µl |
| 10mM dNTPmix | 2 µl |
| 20 µM 5'-IL-5 primer | 1 µl |
| 20 µM 3'-IL-5 primer | 1 µl |
| 5 U/µl Taq polymerase | 0.4 µl |
| sample | 5 µl |
| Total | 50 µl |

**Table 3**

| Step 1 | |
|---|---|
| 95°C | 2:30 |
| 60°C | 2:00 |
| 72°C | 3:00 |

| Step 2-30 | |
|---|---|
| 95°C | 1:00 |
| 60°C | 2:00 |
| 72°C | 3:00 |

### (3) Culture of cancered T cell BUC cell

BUC cells were cultured in RPMI 1640 medium containing 10 % FCS.

### (4) Cell fusion and production of hybridoma

Cell fusion was carried out by the cell fusion method as described in "Zoku-Jikken Seikagaku Koza (Sequel to Biochemistry Experimental Course)", Vol. 5. Immunobiochemistry Research Method (Tokyo Kagakudojin), p. 262 with a slight modification. When the fusion was carried out without stimulating T cell clones as obtained in (2), no hybridoma production was observed (three times repeated). In order to enhance the efficiency of the fusion, T cell clones were then stimulated. One to four days after stimulation with PMA + 10M (PMA, 20nM; I0M, 1µM), cell fusion was carried, and the production of a hybridoma was checked. As the result, the production of the hybridoma was observed when the fusion was carried out 4 days after the stimulation (cf. Table 4). Namely, 1 x 10⁶ of T cell clones 4 days after PMA + I0M stimulation and 10⁷ of BUC cells cultured in a RPMI 1640 medium containing 10 % FCS were mixed together and centrifuged at 1,200 rpm for 10 minutes at 20°C, followed by elimination of the supernatant. Then, 50 % PEG 4,000 solution (Sigma) was added thereto for cell fusion. The fused cells were washed twice with an RPMI 1640 medium free of FCS and cultured on a HAT medium. About one month later, the appearance of 19 hybridomas was observed.

**Table 4**

| | Number of day(s) after stimulation | | | | |
|---|---|---|---|---|---|
| | 0 | 1 | 2 | 3 | 4 |
| Number of hybridomas appeared | 0 | 0 | 0 | 0 | 19 |

### (5) Selection of clones expressing IL-5 mRNA

T cell hybridomas as obtained in (4) were cultured in a 24 well plate (manufactured by CORNING Co.) at a concentration of 2 × 10⁵ cell / well and stimulated with calcium ionophore (PMA, 500 nM; IOM, 1 µM). (When stimulated with PMA 20 nM + I0M 1uM, expression of IL-5 mRNA was not observed in all the clones.) Six hours later, T cell clones were collected. They were centrifuged at 4,000 rpm for 10 minutes at 4°C by the aid of a cooling centrifuge (KUBOTA 1710). After aspiration of the supernatant, 1 ml of ISOGENE (manufactured by Nippon Gene Co.) was added thereto, followed by stirring well. Homogenization was carried out ten times with a 5 ml injection cylinder fitted with a 25G needle. After addition of 200 µl of chloroform and stirring well, centrifugation was carried out at 13,000 rpm for 15 minutes at 4°C. The supernatant (aqueous layer) was collected, 500 ul of isopropanol was added thereto, and stirring was carried out, followed by keeping at -20°C for one hour or more. Centrifugation was carried out at 300 rpm for 15 minutes at 4°C, followed by decantation, and 1 ml of 80% ethanol was added thereto, followed by stirring. Centrifuging at 13,000 rpm for 15 minutes at 4°C, the supernatant was removed by decantation, and the solid material was dried at room temperature. 400 µl of TE buffer, 40 µl of 3M sodium acetate solution (pH 5.8) and 1 ml of 100% ethanol were added thereto, followed by stirring, and then the resultant mixture was kept at -20°C for one hour or more. Additional centrifugation was carried out at 13,000 rpm for 15 minutes at 4°C, the supernatant was removed by decantation, and the solid material was dried at room temperature and dissolved in 10 µl of distilled water. The resulting solution was incubated at 70°C for 4 minutes and cooled on ice. To 10.5 µl of the incubated solution, 9.5 µl of the RT reaction solution as shown in Table 1 was added, followed by reacting at 42°C for 3 hours to transform into cDNA. After completion of the reaction, the PCR reaction solution as shown in Table 2 was prepared, and 45 µl of such solution was added to 5 µl of the cDNA solution. After addition of 33 µl of light mineral oil (manufactured by Sigma Co.), the PCR method was carried out according to the protocol as shown in Table 3. After completion of the PCR method, 2 ul of Type III pigment was added to 10 µl of each of the samples, and electrophoresis was carried out with 1 % gel. Then, the band on electrophoresis was observed. In this manner, the hybridoma clone SA-7 expressing IL-5 m RNA was obtained.

### Example 2: Inhibition of expression of IL-5 mRNA by dexamethasone (DX)

T-cell hybridomas were cultured in a 24 well plate (manufactured by CORNING Co.) at a concentration of 2 × 10⁶ cell/well and stimulated by calcium ionophore (PMA, 500 nM; IOM, 1 µM) while simultaneous addition of 10 nM, 100 nM or 1 µM of DX. After six hours, the T-cell clones were collected and centrifuged at 4,000 rpm for 10 minutes at 4°C by the aid of a cooling centrifuge (KUBOTA 1710). After aspiration of the supernatant, 1 ml of ISOGENE (manufactured by Nippon Gene Co.) was added thereto, followed by stirring well. Homogenization was carried out ten times by the use of a 5 ml injection cylinder fitted with a 25G needle. Two hundred µl of chloroform was added thereto, stirring was effected well, and centrifugation was carried out at 13,000 rpm for 15 minutes at 4°C. The supernatant (aqueous layer) was collected, 500 µl of isopropanol was added thereto, and stirring was effected, followed by keeping at -20°C for one hour or more. Centrifugation was perfomed at 13,000 rpm for 15 minutes at 4°C, the supernatant was removed by decantation, and 1 ml of 80 % ethanol was added thereto, followed by stirring. Another centrifugation was performed at 13,000 rpm for 15 minutes at 4°C, the supernatant was removed by decantation, and the solid material was dried at room temperature. 400 µl of TE buffer, 40 µl of 3M sodium acetate solution (pH 5.8) and 1 ml of 100 % ethanol were added thereto. After stirring well, the resultant mixture was kept at -20 C for one hour or more. A further centrifugation was perform at 13,000 rpm for 15 minutes at 4°C, and the supernatant was removed by decantation, followed by drying at room temperature. The dried product was dissolved in 10 µl of distilled water, incubated at 70°C for 4 minutes and cooled on ice. To 10.5 µl of the resultant solution, 9.5 µl of the RT reaction solution as shown in Table 1 was added, followed by reacting at 42°C for 3 hours to transform into cDNA. After completion of the reaction, the PCR reaction solution as shown in Table 2 was prepared. To 5 µl of the solution containing cDNA, 45 µl of the PCR reaction solution was added. 33 µl of light mineral oil (manufactured by Sigma Co.) was added thereto, and the PCR method was carried out according to the schedule as shown in Table 3. After completion of the PCR method, 2 µl of Type III pigment was added to 10 µl of each sample. Thereafter, electrophoresis was carried out using 1 % gel, and the band on electrophoresis was observed. DX significantly inhibited expression of IL-5 mRNA in 1 µM.

### Example 3: Inhibition of expression of IL-5 mRNA by FK506

A T cell hybridoma was cultured in a 24 well plate (manufactured by CORNING Co.) in the concentration of 2 × 10⁶ cell / well and stimulated by calcium ionophore (PMA 500 nM, IOM, 1 µM) with adding simultaneously 10 nM, 100 nM or 1 µM of FK506. Six hours later, T cell clones were collected. They were centrifuged by a cooling centrifuge (KUBOTA 1710) at 4,000 rpm for 10 minutes at 4°C. After aspirating the supernatant, 1 ml of ISOGENE (manufactured by Nippon Gene Co.) was added, followed by stirring fully. They were homogenized ten times with a 5 ml injection cylinder fitted with a 25G needle. Two hundred µM of chloroform was added and they were centrifuged at 13,000 rpm for 15 minutes at 4°C after stirring fully.

The supernatant (an aqueous layer part) was collected, and 500 µl of isopropyl alcohol was added thereto them. After stirring, they were preserved at -20°C for an hour or more. After centrifuging at 13,000 rpm for 15 minutes at 4°C, the supernatant was decanted and 1 ml of 80 % ethanol was added to them, and followed by stirring. After centrifuging at 13,000 rpm for 15 minutes at 4°C, the supernatant was decanted. After drying at room temperature, 400 µl of TE buffer, 40 µl of 3M sodium acetate solution (pH 5.8) and 1 ml of 100 % ethanol were added to them. After stirring fully, they were preserved at -20°C for an hour or more. After centrifuging at 13,000 rpm for 15 minutes at - 4°C, the supernatant were decanted. After drying at room temperature, they were dissolved in 10 µl of distilled water. After incubating at 70°C for 4 minutes, they were cooled on ice. To 10.5 µl of the resultant solution was added 9.5 µM of the RT reaction solution shown in Table 1, and followed by reacting them at 42°C for 3 hours to transform into cDNA. After completion of reaction, the PCR reaction solution shown in Table 2 was prepared. To 5 µl of cDNA was added 45 µl of the PCR reaction solution. To the solution, 33 µl of Light mineral oil (manufactured by Sigma Co.) was added and the PCR method was carried out according to the schedule shown in Table 3. After completion of the PCR method, 2 µl of Type III pigment was added to 10 µl of each sample. Thereafter, electrophoresis was carried out using 1 % gel. After that, the electrophoresis band was observed. FK506 significantly inhibited mRNA expression of IL-5 in 1 µM.

### Example 4: Introduction of IL-5 gene to hybridoma and its expression

An IL-5 gene is cloned and it (NF-AT-IL-5) was incorporated into a basic vector having luciferase activity (manufactured by Nippon Gene Co.) and a p515 IL-5-luc was cut away. It was introduced to a hybridoma of the present invention by Electroporation method. Namely, using an apparatus for introducing gene, GTE-10 (manufactured by Shimazuseisakusho Co.) at 250 V and 800 µF, it was introduced to the hybridoma in the concentration of 1 × 10⁶ cell / 0.5 ml / cell. After the introduction, it was stimulated by calcium ionophore (PMA, 20 nM; IOM, 1 µM). Twenty-four hours later, hybridomas were collected. They were centrifuged by a cooling centrifuge at 4,000 rpm for 10 minutes at 4°C. After aspiration of the supernatant, a cell solubilizing solution was added, and the resultnat mixture was allowed to stand for 10 minutes at room temperature. After centrifuging for about 5 seconds, the supernatant was removed and the substrate was added thereto, followed by measurement of the luminous intensity by a scintillation counter. By PMA + IOM stimulation, the luciferase activity increased about 50 times.

### Example 5: Introduction of IL-5 gene to hybridoma and inhibition of expression thereof

A p515 IL5-luc gene was introduced into the hybridoma of the invention by the electroporation method. That is, using an apparatus for introduction of a gene (manufactured by Shimazuseisakusho Co.), introduction into the hybridoma was carried out with a concentration of 1 × 10⁶ cell / 0.5 ml / cell at 250 V and 800 µF. After completion of the introduction, stimulation by calcium ionophore (PMA, 20 nM; IOM, 1 µM) was effected with simultaneous addition of 10 nM - 1 µM of DX or FK506. Twenty-four hours later, hybridomas were collected. They were centrifuged by a cooling centrifuge (KUBOTA 1710) at 4,000 rpm for 10 minutes at 4°C. After aspiration of the supernatant, a cell solubilizing solution was added thereto, followed by allowing to stand for 10 minutes at room temperature. After centrifugation for about 5 seconds, the supernatant was removed, and the substrate was added, followed by measurement of the luminous intensity by a scintillation counter. Luciferase activity after stimulation by PMA + 10M was measured, and it was confirmed that the luciferase activity increased about 50 times by such stimulation. On the other hand, addition of 1 µM of FK506 resulted in decrease of said activity to 30 %. From these results, it is understood that FK506 acts on the NF-AT part. In case of DX, its addition in 1 µM did not produce any decrease of the activity.

### Brief description of drawing:

Fig. 1 is a drawing wherein an IL-5 gene is incorporated into a plasmid vector.

## Claims

1. A hybridoma capable of expressing mRNA of interleukin-5 (IL-5) but not producing IL-5, which is obtained by fusing a human T cell clone expressing IL-5 mRNA with a cancered T cell.

2. A hybridoma according to claim 1, which is transformed with a plasmid comprising an IL-5 gene and an enzyme gene incorporated therein.

3. A hybridoma according to claim 2, wherein the enzyme gene is a luciferase gene.

4. A method for screening an antiasthmatic or antiallergic drug, which comprises activating a hybridoma according to claim 1 in the presence of a compound or substance to be tested and observing whether the expression of the IL-5 gene takes place.

5. A method for screening an antiasthmatic or antiallergic drug, which comprises activating a hybridoma according to claim 2 in the presence of a compound or substance to be tested and then observing whether the expression of the IL-5 gene takes place indirectly through measurement of the enzyme activity.
